# EUROPÄISCHE PATENTSCHRIFT

(11) **EP 3 824 797 B1**
(45) Veröffentlichungstag und Bekanntmachung des Hinweises auf die Patenterteilung: **27.09.2023**
(21) Anmeldenummer: 20174551.0
(22) Anmeldetag: 14.05.2020
(51) Int. Cl.: A61B 3/06, A61B 3/024, A61B 3/032

(54) **VERFAHREN UND VORRICHTUNG ZUR AKQUISITION VON DATEN, DIE SICH AUF EINE WAHRNEHMUNG VON FARBEN DURCH EINE TESTPERSON BEZIEHEN**
METHOD AND DEVICE FOR ACQUIRING DATA RELATING TO PERCEPTION OF COLOURS BY A TEST PERSON
PROCÉDÉ ET DISPOSITIF D'ACQUISITION DES DONNÉES RELATIVES À LA PERCEPTION DE COULEURS PAR UN COBAYE HUMAIN

(30) Priorität: 22.11.2019 DE 102019131679
(43) Veröffentlichungstag der Anmeldung: 26.05.2021
(73) Patentinhaber: Hochschule Aalen, 73430 Aalen (DE); Universität Heidelberg, 69117 Heidelberg (DE)
(72) Erfinder: Schiefer, Ulrich, 72127 Kusterdingen (DE); Krastel, Hermann, 69151 Neckargemünd (DE); Ungewiß, Judith, 73430 Aalen (DE); Wörner, Michael, 70199 Stuttgart (DE)
(74) Vertreter: DREISS Patentanwälte PartG mbB

(56) Entgegenhaltungen:
- WO-A2-2011/016029
- DE-A1-102019 121 602
- US-A- 5 946 075
- US-A1- 2003 081 176
- US-A1- 2008 024 724

## Beschreibung

Die vorliegende Erfindung betrifft ein Verfahren zur Akquisition von Daten und eine entsprechende Testvorrichtung. Die Daten beziehen sich auf eine Wahrnehmung von Farben durch eine Testperson. Ein diesem Zweck dienendes Verfahren und eine entsprechende Testvorrichtung sind aus der WO2011/016029 A2 bekannt. Die bekannten Gegenstände arbeiten mit einem Pupillometer, das unbewußt erfolgende Reaktionen von Pupillen von Testpersonen auf Lichtreize erfasst.

Derartige Daten erlauben eine Quantifizierung von Farbsinndefekten/-störungen, speziell im langwelligen Spektralbereich, einschließlich der Protanopie ("Rotblindheit") und Protanomalie ("Rotschwäche").

Hereditäre, X-chromosomal vererbte Farbsinnstörungen sind durch eine funktionelle Störung des betroffenen Zapfensystems gekennzeichnet, die zu einer gestörten Farbwahrnehmung führt. Der Erbgang führt dazu, dass überwiegend männliche Personen (in Deutschland ca. 8% aller Männer) und nur selten Frauen (nämlich nur im Falle eines genetischen Defekts auf *beiden* X-Chromosomen,) betroffen sind.

Im Falle einer Funktionsstörung der langwelligen (L- = Rot-)Zapfen fällt dieser Rezeptortyp entweder komplett (Protanopie, in Deutschland ca. 1% aller Männer) oder partiell (Protanomalie, in Deutschland ebenfalls ca. 1% aller Männer) aus. Da das spektrale Empfindlichkeitsprofil der L-Zapfen den roten Randbereich des wahrnehmbaren Spektrums repräsentiert, kommt es bei Protan-Störungen zu einer subjektiven Minderung der Helligkeitswahrnehmung bei langwelligen (roten) Farbreizen. Dies hat u. a. die Auswirkung, dass von Personen mit einer Rotsinnstörung Rücklichter und / oder Bremslichter u. U. später erkannt werden als von Normalpersonen.

In der konventionellen klinischen Funktionsdiagnostik erfolgt eine Akquisition von derartigen Farbsinnstörungen betreffenden Daten mittels Farbtafelsystemen oder kleinflächigen Farbreizen ("Farbtöpfchen") unter Ausnutzung von spezifischen Farbverwechslungen. Farbsinnstörungen werden mit einem sogenannten Anomaloskop guantifiziert.

Hier wird ein kleinflächiger, geteilter Farbreiz dargeboten:
Hierbei zeigt ein Halbfeld ein spektral reines, durch ein spezielles Farbfilter erzeugtes Vergleichs -/ Referenzfeld an, dessen Leuchtdichte variiert werden kann. Das andere Halbfeld wird durch das Mischungsverhältnis zweier Farbreize erzeugt, welche zwei benachbarte Zapfentypen ansprechen: Im Falle der Diagnostik von Funktionsstörungen im Bereich der mittelwellige n (M- = Grün) und der benachbarten langwelligen (L- = Rot) Zapfen hat das (in der Leuchtdichte variable) Referenz-Halbfeld die Farbe "gelb" und das obere Halbfeld wird durch das Mischen der Farben "grün" und "rot" erzeugt.

Eine Person mit einer Protan- (Rotsinn-)Störung wird im oberen Halbfeld (im Gegensatz zur Normeinstellung normaler Farbsichtiger = normaler Trichromaten) einen zu geringen Rotanteil empfinden, das Feld als "grünlicher" empfinden als eine farbtüchtige Normalperson und daher im oberen Feld mehr Rotanteile zumischen. Aufgrund des Defekts seiner Rot-Rezeptoren wird er das obere Feld zudem "dunkler" empfinden und das untere Vergleichsfeld auch in seiner Leuchtdichte ("Helligkeit") reduzieren. Dieser "Helligkeitsverlust" ist umso ausgeprägter, je mehr Rotanteile im oberen Vergleichsfeld hinzugemischt werden müssen, die ein rotsinngestörter Patient aufgrund der fehlerhaften Rot-Zapfenfunktion als "dunkler" empfindet.

Verschiebungen des Rot-/Grün-Mischungsverhältnisses werden durch den sog. "Anomalie-Quotienten (AQ)" quantifiziert. Dieser liegt für farbtüchtige Normalpersonen bei ca. 1,0 (0,7 bis 1,3), bei Patienten mit einer Rotschwäche (Protanomalie) liegt der AQ unterhalb von 0.7; von einer ausgeprägten Protanomalie wird bei einem AQ unterhalb von 0.5 gesprochen. Im Falle einer Rotblindheit (Protanopie) findet der Patient keine einzelne Zone des optimalen Mischungsverhältnisses im oberen Mischungshalbfeld und im unteren Referenzvergleichsfeld.

Vielmehr kann er für jedes Rot-/Grün-Mischungsverhältnis eine zugehörige (Leuchtdichte-)Einstellung des unteren Referenzfelds finden, die für ihn als gleich hell und zugleich auch als gleichfarbig empfunden wird: Je weiter das Mischungsverhältnis in den "roten"/langwelligen Spektralbereich wandert, desto "dunkler" wird das Referenzfeld gedimmt. Somit lässt sich ausgerechnet die ausgeprägteste Form der Rotsinnstörung, die Protanopie, nicht durch einen zugehörigen Anomalie-Quotient (AQ) quantifizieren.

Erschwerend kommt hinzu, dass die zu mischenden roten und grünen Farbreize des oberen "Mischungs-Halbfelds" ausgerechnet entlang einer sogenannten "Verwechslungsgeraden" liegen - und der befragte Patient sich häufig nicht sicher ist, welche Farbbezeichnung (rot oder grün) er diesem Mischungs-Halbfeld überhaupt geben soll - nicht selten wechselt patientenseitig die Bezeichnung zwischen "rot" und grün", was die Untersuchung und den Informationsaustausch für alle Seiten erschwert.

Zusammengefasst heißt dies, dass eine mit einem Anomaloskop erfolgende Akquisition von Daten eine relative komplexe Untersuchung darstellt, deren Ergebnisse auf die Gesichtsfeldmitte beschränkt sind und insbesondere beim Vorliegen einer Protanopie ("Rotblindheit") eine Quantifizierung nicht erlauben.

Die Aufgabe der vorliegenden Erfindung besteht in der Angabe eines Verfahrens und einer Testvorrichtung zur Akquisition von auf eine Farbwahrnehmung bezogenen Daten, die eine quantitative Beurteilung von Wahrnehmungsdefiziten von Farben, insbesondere für Farben aus dem roten Spektralbereich des mit dem menschlichen Sehsinn sichtbaren Lichtes auch außerhalb eines zentralen Bereichs des Gesichtsfeldes einer Testperson erlauben.

Diese Aufgabe wird in Bezug auf ihre Verfahrensaspekte durch die Merkmale des Anspruchs 1 und in Bezug auf ihre Vorrichtungsaspekte durch die Merkmale des unabhängigen Vorrichtungsanspruchs gelöst. Bei dem Verfahren handelt es sich bevorzugt um ein computerimplementiertes Verfahren.

Die Methodik basiert auf den Prinzipien einer Gesichtsfelduntersuchung, vorzugsweise einer kinetischen Perimetrie mit bewegten Prüfmarken, wie sie aus der DE 100 13 682 C bekannt ist: Nach dieser Schrift werden die Exzentrizitäten der individuellen, Reaktionszeit-korrigierten, lokalen Wahrnehmungsschwellen für mit einer Winkelgeschwindigkeit von 4°/s bewegte Messmarken ermittelt.

Das erfindungsgemäße Verfahren zeichnet sich durch die folgenden Schritte aus. Es wird eine Testvorrichtung bereitgestellt, die eine Präsentationsfläche aufweist, auf der visuelle Stimuli in variablen Positionen und wenigstens eine ortsfeste Blickfixierungsmarkierung darstellbar sind. Dabei weist die Testvorrichtung eine Eingabevorrichtung auf, mit der Reaktionen der Testperson erfassbar und aufzeichnungsbar sind.

Die Testperson wird so platziert, dass die Blickfixierungsmarkierung in einem zentralen Bereich des Sichtfeldes der Testperson liegt. Die Testperson wird angewiesen, ihren Blick auf die Blickfixierungsmarkierung zu richten und nicht von der Blickfixierungsmarkierung zu lösen. Die Testperson wird weiter angewiesen, eine bestimmte Reaktion zu zeigen, wenn sie einen visuellen Stimulus auf der Präsentationsfläche wahrnimmt.

Visuelle Stimuli werden entlang einer vorbestimmten Bahnkurve in verschiedenen Positionen auf der Präsentationsfläche präsentiert. Die Bahnkurve ist bevorzugt eine Gerade. Die Bewegung eines Stimulus erfolgt dann linear entlang von Vektoren, die durch Ursprung (Vektor-/Pfeil-Beginn) und Ende (Pfeilspitze) gekennzeichnet sind.

Weiter wird die Testperson auf das Zeigen der bestimmten Reaktion überwacht. In dem Moment, in dem die Testperson die Reaktion zeigt, werden die Daten, welche die Positionen der visuellen Stimuli kennzeichnen, erfasst und gespeichert.

Aus den gespeicherten Daten wird ein Grenzpunkt der Bahnkurve ermittelt, der eine Grenze zwischen Positionen von Stimuli markiert, auf welche die Testperson reagiert, und - von der Blickfixierungsmarkierung aus betrachtet - weiter außen liegenden Positionen von Stimuli, auf welche die Testperson noch nicht reagiert.

Es wird ein Maß für das Gesichtsfeld der Testperson in Abhängigkeit von dem Grenzpunkt ermittelt, wobei das Maß als ein erstes Maß für erste Stimuli ermittelt wird, die farblos sind oder eine erste Farbe aufweisen, und wobei das Maß als ein zweites Maß für zweite Stimuli ermittelt wird, die eine zweite Farbe aufweisen. Die sich auf eine Wahrnehmung von Farben durch eine Testperson beziehenden Daten werden durch Normierung des zweiten Maßes auf das erste Maß gebildet.

Mit diesen Merkmalen lassen sich Wahrnehmungsdefizite insbesondere für langwelliges (rotes) Licht im gesamten Gesichtsfeld ermitteln und mit der Fähigkeit zur Wahrnehmung weißer, bzw. farbloser Stimuli vergleichen. Im Gegensatz zur Akquisition von Daten mit dem Anomaloskop ist das erfindungsgemäße Verfahren einfacher durchführbar, nicht auf die Gesichtsfeldmitte beschränkt, und es erlaubt eine Quantifizierung auch beim Vorliegen einer Protanopie ("Rotblindheit").

Die Erfindung lässt sich durch Abwandlung eines (verfügbaren) perimetrischen Verfahrens durch Einsatz von Rotfiltern verwirklichen. Ein wesentlicher Vorteil besteht demnach auch darin, dass ein Perimeter (im Gegensatz zum Anomaloskop) in praktisch jeder Arztpraxis zur Verfügung steht. Die perimetrische Akquisition von Daten kann zudem an (versiertes) Fachpersonal delegiert werden. Die Untersuchungsergebnisse sind leicht vermittelbar und nachvollziehbar und könnten ggf. gutachterliche Relevanz erhalten.

Eine bevorzugte Ausgestaltung zeichnet sich dadurch aus, dass der Stimulus mit vorgegebener Geschwindigkeit kontinuierlich entlang einer Bahnkurve bewegt wird. Wie weiter oben erwähnt, ist das Verfahren auch mit örtlich diskreten, statischen Darstellungen durchführbar. Dann erfolgt eine Abfolge räumlich diskreter Darstellungen mit vorgegebener Schrittgeschwindigkeit.

Bevorzugt ist auch, dass als visueller Stimulus ein Lichtfleck verwendet wird, der von der Testperson aus unter einem Sehwinkel von 3° erscheint.

Weiter ist bevorzugt, dass die zweite Farbe Rot ist.

Eine weitere bevorzugte Ausgestaltung zeichnet sich dadurch aus, dass es für verschiedene Leuchtdichten und Größen (Sehwinkel) der visuellen Stimuli sowie andere Eigenschaften (z.B. Flackern)(14) durchgeführt wird.

Bevorzugt ist auch, dass die visuellen Stimuli mit vorgegebener Geschwindigkeit kontinuierlich entlang einer Bahnkurve bewegt werden, die durch die Blickfixierungsmarkierung verläuft.

Weiter ist bevorzugt, dass aus den längs jeweils einer Bahnkurve erfassten Daten durch statistische Bewertung wie Mittelwertbildung oder Medianbildung von Schwellenwertabständen für diese Bahnkurve ein Grenzpunkt bestimmt wird, dessen Abstand vom Schnittpunkt für diese Bahnkurven eine Entfernung kennzeichnet, ab dem der Stimulus für die betreffende Testperson erkennbar ist. Jeder Schwellenwertabstand kennzeichnet einen Abstand von der Blickfixierungsmarkierung für diese Bahnkurve innerhalb dem die betreffende Testperson den visuellen Stimulus erkannt hat.

Der Schwellenwertabstand ist dabei der Abstand des Stimulus von dem Bahnkurvenursprung bei einem einmaligen Durchlaufen der Bahnkurve. Ggf. werden mit einem mehrmaligen Durchlaufen der Bahnkurve mit gleichen Stimuli mehrere Schwellenwertabstände bestimmt, die dann statistisch ausgewertet werden.

Eine weitere bevorzugte Ausgestaltung zeichnet sich dadurch aus, dass bei der Bestimmung der Daten die Reaktionszeit der Testperson berücksichtigt wird.

Bevorzugt ist auch, dass die Berücksichtigung dadurch erfolgt, dass der Teil der Bahnkurve, den der Stimulus in der Reaktionszeit zurückgelegt als Produkt aus Reaktionszeit und Stimulusgeschwindigkeit bestimmt wird und dass der zunächst ohne Berücksichtigung der Reaktionszeit bestimmte Punkt der Bahnkurve um diesen Teil der Bahnkurve in Richtung zu der initialen Position (in Gegenrichtung der Stimulusbewegung) verlegt wird.

Weiter ist bevorzugt, dass die Schrittfolge des Anspruchs 1 beendet wird, wenn sich der Blick der Testperson von der Blickfixierungsmarkierung löst und alle ggf. bereits für diesen Stimulus festgestellten Wahrnehmungen/Reaktionen verworfen werden.

Eine weitere bevorzugte Ausgestaltung zeichnet sich dadurch aus, dass das Darstellen eines Stimulus für eine vorbestimmte Präsentationsdauer erfolgt und dass dann, wenn innerhalb der Präsentationdauer keine Reaktion der Testperson erfolgt, für die betreffende Präsentationsdauer auch keine Daten akquiriert werden.

Weitere Vorteile ergeben sich aus der nachfolgenden Beschreibung, den Zeichnungen und den Unteransprüchen. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Ausführungsbeispiele der Erfindung sind in den Zeichnungen dargestellt und werden in der nachfolgenden Beschreibung näher erläutert.

Dabei zeigen, jeweils in schematischer Form:
- Figur 1: ein Ausführungsbeispiel einer erfindungsgemäßen Vorrichtung;
- Figur 2: eine grafische Veranschaulichung von mit der Erfindung akquirierbaren Daten bei einem normalen Trichromaten;
- Figur 3: eine grafische Veranschaulichung von mit der Erfindung akquirierbaren Daten bei einem Protanomalen; und
- Figur 4: ein Flussdiagramm als Ausführungsbeispiel eines erfindungsgemäßen Verfahrens.

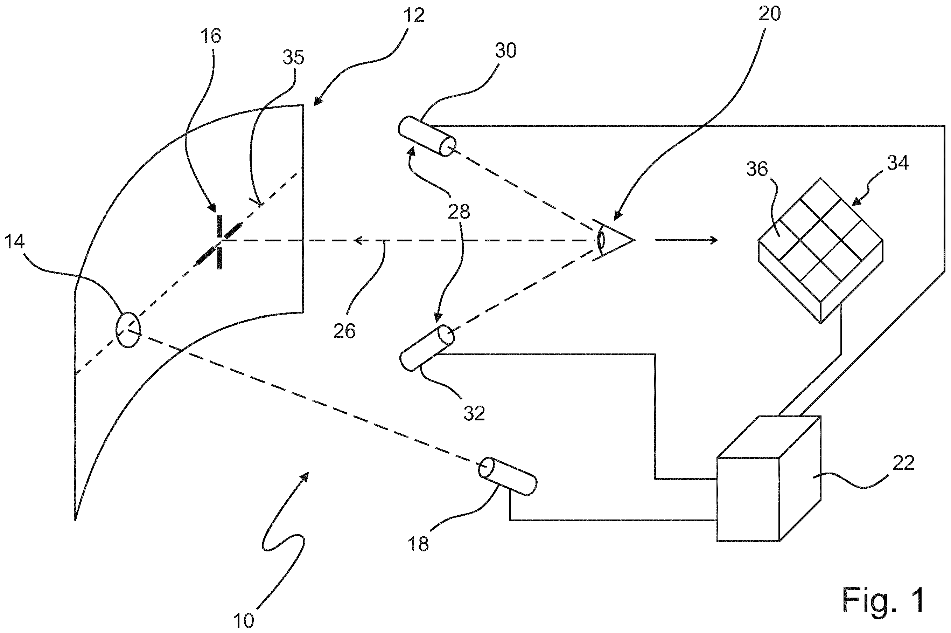

Im Einzelnen zeigt die Figur 1 eine Testvorrichtung 10 mit einer Präsentationsfläche 12, auf der visuelle Stimuli 14 in variablen Positionen zusammen mit einer ortsfesten Blickfixierungsmarkierung 16 darstellbar sind.

Die Stimuli 14 sind in Bezug auf ihre Farbe, Größe (Sehwinkel) und Helligkeit (Leuchtdichte), Winkelgeschwindigkeit und ihre Helligkeitsänderung (z.B.: "Flackern") variabel.

Die Präsentationsfläche 12 kann zum Beispiel eine kuppelförmige Projektionsfläche sein, auf der die Stimuli 14 in variablen Positionen und Helligkeiten zusammen mit wenigstens einer ortsfesten Blickfixierungsmarkierung 16 darstellbar sind. In einer bevorzugten Ausgestaltung dient ein Projektor 18 zur Projektion von Stimuli 14 auf die Präsentationsfläche 12. Die Präsentationsfläche 12 kann aber auch als ein Bildschirm eines elektrischen Bildschirmgerätes verwirklicht sein. Die Präsentationsfläche 12 ist bevorzugt um den Ort 20, an dem sich die Testperson während der Akquisition der Daten befindet, herum gewölbt. Der Projektor 18, bzw. das elektrische Bildschirmgerät, wird von einem Steuergerät 22 der Testvorrichtung 10 gesteuert.

Die Blickfixierungsmarkierung 16 kann ein stofflicher Bestandteil der Präsentationsfläche 12 sein oder von einem Projektor, der mit dem die Stimuli projizierenden Projektor 18 identisch sein kann, auf die Präsentationsfläche 12 projiziert werden. Der Projektor weist eine Anordnung von mehreren Leuchtdioden, bevorzugt eine Anordnung von RGB-Leuchtdioden als Lichtquelle auf, mit denen bekanntlich Licht mit variabler Lichtfarbe erzeugbar ist. Bei Verwendung eines Bildschirmgerätes wird die Blickfixierungsmarkierung 16 auf dem Bildschirm erzeugt.

Eine Variation der Farbe und/oder Größe und/oder Helligkeit der Stimuli 14 kann alternativ oder ergänzend auch durch vorgeschaltete Filter- und Diffusorelemente/- folien erreicht werden.

Die Stimuli 14 werden entweder in diskreten Schritten bzw. an definierten Orten (Raster) dargestellt (*statische* Untersuchungsmethode)oder von außerhalb des Gesichtsfeldes kommend nach innen zur Blickfixierungsmarkierung 16 oder kontinuierlich bewegt (*kinetische* Untersuchungsmethode). Beim Unterschreiten eines Schwellenabstandes werden die Stimuli 14 von der Testperson wahrgenommen, was eine Reaktion der Testperson auslöst. Die Reaktion der Testperson löst wiederum eine Speicherung der Position des Stimuli 14 aus. Die gespeicherte Position gibt einen Wahrnehmungs-Schwellenabstand an. Die Erfindung erlaubt eine ortsaufgelöste Bestimmung solcher Schwellenabstände z.B. dadurch, dass in einem Raster von {bevorzugt konzentrisch) um die Blickfixierungsmarkierung herum angeordneten Positionen mindestens ein Stimulus 14 eingeblendet wird, das definierte Eigenschaften wie Sehwinkel {d.h. Größe) und Kontrast besitzt. Diese Schwellenabstände sind Beispiele von erfindungsgemäß zu akquirierenden Daten. Anstelle einer Erzeugung von Stimuli an Punkten eines flächigen Rasters werden die Stimuli bevorzugt längs eines horizontalen Meridians der Projektionsfläche 12 erzeugt.

Eine Blickrichtungserkennungsvorrichtung 28 (eye tracking System) umfasst zwei Kameras 30, 32, die beide auf den genannten Ort 20 ausgerichtet sind und ihre Bildsignale an das Steuergerät 22 übermitteln. Das Steuergerät 22 ist dazu eingerichtet, durch eine Bildauswertung die Blickrichtung 26 und insbesondere Änderungen der Blickrichtung 26 einer Testperson, die sich am genannten Ort 20 befindet, zu ermitteln.

Die Blickrichtungserkennungsvorrichtung 28 umfasst damit die beiden Kameras 30, 32 und das Steuergerät 22. Eye tracking Systeme und Details der Bildauswertung werden hier als bekannt vorausgesetzt. Eine für das Beibehalten der Blickrichtung 26 der Testperson hilfreiche Positionierung des Kopfes der Testperson kann ggf. durch eine Kinn-/Stirnstütze zusätzlich verbessert werden.

Die erfindungsgemäße Testvorrichtung 10 weist darüber hinaus eine Eingabevorrichtung 34 auf, die dazu eingerichtet ist, Reaktionen der Testperson zu erfassen und zur Aufzeichnung an das Steuergerät 22 zu übergeben. Das eine Reaktion auslösende Ereignis ist zum Beispiel die Wahrnehmung eines Stimulus 14 auf der Präsentationsfläche 12 durch die Testperson. Die Reaktion besteht zum Beispiel darin, dass die Testperson den Ort des Stimulus 14 auf der Präsentationsfläche 12 angibt. In einem Beispiel kann die Eingabevorrichtung 34 eine Matrix von Tasten 36 aufweisen, von denen jede einen bestimmten Bereich auf der Präsentationsfläche 12 repräsentiert. Die Testperson gibt den Bereich oder Ort, in dem sie den Stimulus wahrgenommen hat, durch Betätigen der diesem Bereich zugeordneten Taste 36 an. Bei einer Messung, die nur entlang einer einzigen Bahnkurve, bspw. entlang eines horizontalen Meridians erfolgt, reicht auch eine Eingabevorrichtung mit nur einer einzigen Taste 36 aus. Da das Steuergerät 22 die Position des Stimulus 14 zu jedem Zeitpunkt kennt, reicht eine durch die Reaktion definierte Zeitangabe zur Festlegung der Wahrnehmungsposition, bzw. des Schwellenabstandes aus.

In einer anderen Ausgestaltung wird die Testperson aufgefordert, den Ort und /oder Wahrnehmungszeitpunkt wahrgenommener Stimuli in vorgegebenen Stichworten zu beschreiben, die von Messpersonal der Testvorrichtung 10 oder von einer Spracherkennung, die z.B. ebenfalls durch das Steuergerät 22 erfolgt, aufgenommen werden.

Dadurch wird im Dialog mit der Testperson für jede Position eines auf der Präsentationsfläche 12 dargestellten Stimulus 14 festgestellt, ob die Testperson den eingeblendeten Stimulus 14 wahrnimmt.

In Bezug auf die Verfahrensaspekte erfolgt sequenziell ein Darstellen von wenigstens einem Stimulus 14 an mehreren in dem Sehbereich der Testperson liegenden Positionen, wobei die Darstellung jeweils für eine vorbestimmte Präsentationsdauer erfolgt. Eine Darstellungssequenz beginnt jeweils außerhalb des Gesichtsfeldes der Testperson. Anschließend wird der laterale Abstand des Stimulus 14 zur Blickfixierungsmarkierung sukzessive in diskreten Schritten oder bevorzugt kontinuierlich verkleinert, so dass sich der Stimulus 14 allmählich in das Gesichtsfeld der Testperson hineinbewegt und, je nach Testperson, früher oder später (d. h. bei kleineren oder größeren Schwellenabständen zur Blickfixierungsmarkierung) von der Testperson wahrgenommen werden kann.

Die Testperson reagiert auf eine solche Wahrnehmung mit der Betätigung der Eingabevorrichtung 34.

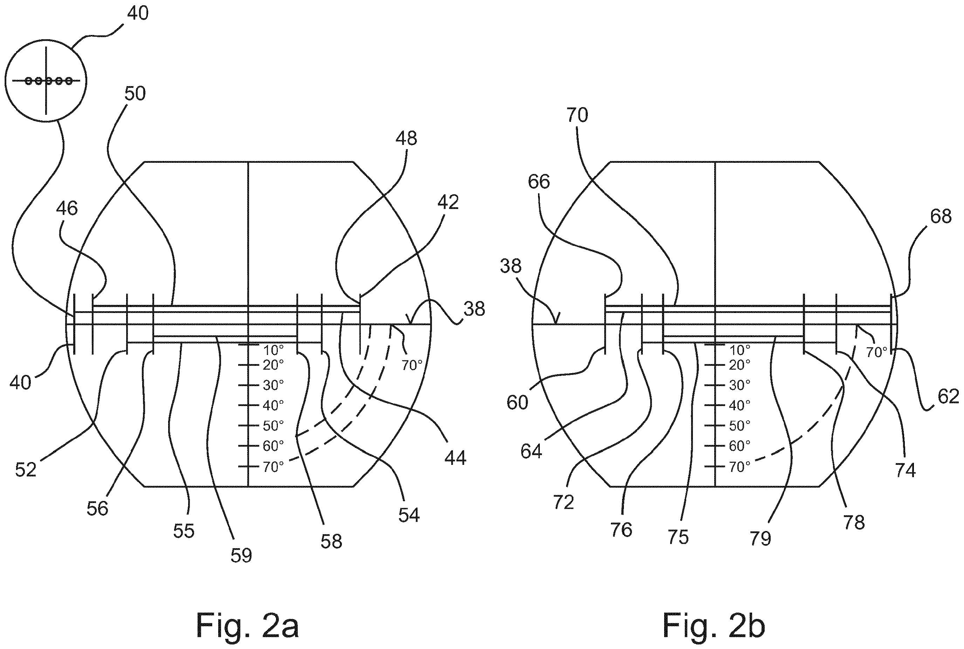

Dem Steuergerät 22, das die Darstellung der Stimuli 14 steuert, sind die jeweiligen Positionsdaten des Stimulus 14 bekannt. Das Steuergerät 22 speichert die aktuellen Positionsdaten jeweils dann ab, wenn die Eingabevorrichtung 34 betätigt wird. Dieser Ablauf wird für jeden Stimulus-Typ, der durch Farbe, Größe und Leuchtdichte gekennzeichnet ist, ggf. mehrfach wiederholt. Die Größe definiert dabei den Sehwinkel, unter dem der Stimulus 14 für die Testperson sichtbar ist. Es ergeben sich dadurch zum Beispiel Datenverteilungen, wie sie in der Figuren 2 als Kreiswolke dargestellt sind. Der Mittelpunkt von jedem der Kreise gibt den Ort auf der Präsentationsfläche 12 an, für den die Testperson eine Reaktion auf eine Wahrnehmung eines Stimulus 14 an diesem Ort eingegeben hat. Dieser Ort wird z.B. durch Angabe der Daten charakterisiert, welche die (hier horizontal verlaufende) Bahnkurve 35 und den jeweiligen Schwellenabstand kennzeichnen. Ein solches Wertepaar stellt ein Beispiel erfindungsgemäß akquirierter Daten dar.

Eine Bewertung von Größe und Lage des von Kreisen freien Bereichs zwischen zwei für gleiche Stimuli bestimmten Abstands der Mediane der Kreiswolken erlaubt Rückschlüsse auf eine Testperson-individuelle Beeinträchtigung ihrer Fähigkeit, die durch die Farbe der Stimuli definierten Farben unter standardisierten und reproduzierbaren Testbedingungen wahrzunehmen.

Bei einer statischen Darbietung kann die Präsentationszeit des Stimulus 14, bei der dynamischen Darstellung auch dessen Winkelgeschwindigkeit variiert werden. Mit dem Begriff der Winkelgeschwindigkeit ist hier die auf den Abstand der Testperson von der bevorzugt kuppelförmigen Projektionsfläche normierte "Bahngeschwindigkeit" gemeint, mit der sich der Stimulus auf der Projektionsfläche bewegt.

Al Stimuli 14 kommen bevorzugt Gutachten-relevante weiße Reizmarken (Leuchtdichte: 320 cd/ m² = 4e; Sehwinkel: 26'= GOLDMANN III) und zum anderen reizschwächere weiße Reizmarken (z.B. Leuchtdichte: 6.31 cd/ m² = GOLDMANN 1c; Sehwinkel: 26'= GOLDMANN III) sowie bezüglich der vorgenannten Sehwinkel (und Leuchtdichte-bezogenen Graufiltereinstellungen) identische rote Reizmarken, jeweils in der nasalen und temporalen Peripherie des horizontalen (0° - 180°) Meridians zum Einsatz. Als Rotfilter kann zum Beispiel ein Filter RG 610, Fa. SCHOTT, Mainz/D eingesetzt werden. Optimalerweise wird eine weitgehend präferentielle Stimulation der L-Zapfen durch einen "langwelligen Reiz" mittels eines Filters mit einem short wavelength cut-off [half power] verwendet. Der cut-off kann zum Beispiel bei 645 nm erfolgen.

In einer Ausgestaltung wird für eine Stimulus-Eigenschaft jeweils die maximale Ausdehnung ("Extent" in°) der Stimulus-Wahrnehmung entlang des horizontalen Meridians als Bahnkurve ermittelt.

Alternativ oder ergänzend können auch Ausdehnungen in weiteren Meridianen, Isopterenflächen oder durch mehrere Isopteren spezifizierte Flächen in beliebigen Gesichtsfeldteilbereichen oder im Gesamtgesichtsfeld verglichen werden, die mit den weißen, bzw. farblosen und roten Stimulus-Reizmarkeneigenschaften ermittelt wurden. Der Übergang von einem durch Schwellenabstände auf einer Bahnkurve ausgemessenen eindimensionalen Intervall auf eine Isopterenfläche erfordert eine Messung entlang von mehreren Bahnkurven, bevorzugt von mehreren Meridianen. Die Isopterenfläche ergibt sich dann durch Verbinden der Grenzen von eindimensionalen Intervallen von einander benachbarten Meridianen oder anderen Bahnkurven. Alternativ können derartige Ausdehnungen auch auf einen Meridian, insbesondere auf den für verkehrsgutachterliche Zwecke besonders bedeutungsvollen horizontalen Meridian beschränkt werden.

Mehrfachdarbietungen identischer Stimuli mit gleichen Eigenschaften entlang ein- und desselben Messvektors (bzw. entlang ein- und derselben Bahnkurve) ermöglichen es, das zentrale Maß (z.B. Median) und die Streuung (z.B. "interquartile range" = IQR) der lokalen Wahrnehmungsschwellen zu ermitteln. Durch Anbieten weit überschwelliger Reize entlang sogenannter Reaktionszeit-Messvektoren in intakten Gesichtsfeldbereichen kann die individuelle Reaktionszeit bestimmt werden. Hierdurch ist eine entsprechende Schwellenkorrektur in Bezug auf die Messvektoren möglich. Die sogenannte Rot-/ Weiß-Dissoziations-Ratio (RWR) ist der Quotient der jeweiligen Ausdehnung (hierbei kann der Median oder z.B. das 25. Percentil oder ein anderer definierter Exzentrizitätswert herangezogen werden) für rote und weiße Stimuli identischer Größe.

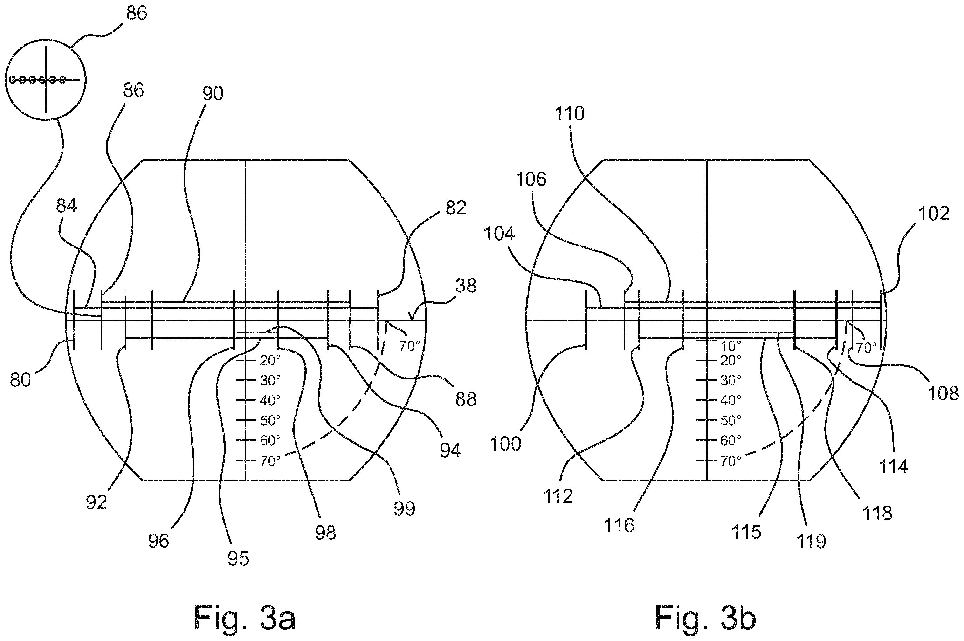

Die Figuren 2 und 3 stellen akquirierte Daten (jeweils seitengetrennt, von rechtem und linkem Auge) bei einem normalen Trichromaten (Figur 2) bzw. bei einem Protanomalen (Figur. 3) dar. Die RWR kann sowohl seitengetrennt, für das rechte Auge und für das linke Auge (hierbei wird das nicht untersuchte Auge durch eine opake Augenklappe abgedeckt), bestimmt werden. Auf diese Weise ist ein inter-okularer Funktionsvergleich möglich. Prinzipiell ist auch eine Ermittlung der RWR für *beide Augen gleichzeitig* (binokulares Gesichtsfeld) möglich. Die RWR kann Werte zwischen O und 1 annehmen: Der Wert "l" besagt, dass es bei Verwendung roter Messmarken - im Vergleich zu weißen Stimuli - zu keinerlei Einschränkung der Gesichtsfeldausdehnung kommt. Je geringer die RWR ist, desto ausgeprägter ist die Einschränkung der (z.B.) horizontalen Gesichtsfeldausdehnung bei Verwendung roter Stimuli im Vergleich zu weißen Reizmarken. Für große, reizstarke (vor allem weiße) Prüfmarken ist, speziell bei jungen, augengesunden Patienten, ein "Deckeneffekt" ("ceiling effect") zu erwarten: Hierbei überschreitet die physiologische Ausdehnung des horizontalen Gesichtsfelds die technischen Grenzen des Geräts. Diese Problematik kann prinzipiell durch einen geeigneten Fixationspunkt-Versatz gelöst werden. Alternativ ist der Einsatz geeigneter, reizschwächerer Reizmarken eine weitere Problemlösungs-Option.

Im Einzelnen zeigt die Figur 2 in ihren Teilen 2a und 2b jeweils längs eines horizontalen Meridians 38 erfasste Abstände von Grenzen des Gesichtsfeldes einer normalsichtigen, keine Rotschwäche aufweisenden Testperson (Trichromat) für vier verschiedene Stimuli. Die Grenzen sind dabei jeweils Mediane von ermittelten Schwellenwertabständen. Die beiden ganz außen liegenden Mediane 40, 42 schließen ein Gesichtsfeld 44 für weiße/farblose Stimuli einer ersten Leuchtdichte/Helligkeit ein. Die Mediane 46 und 48 (fällt mit 42 zusammen) schließen ein Gesichtsfeld 50 für rote Stimuli ein, die in Bezug auf ihre Helligkeit und Größe den weißen/farblosen Stimuli des Gesichtsfeldes 44 entsprechen.

Die durch Quotientenbildung erfolgte Normierung der Breite des Gesichtsfeldes 50auf die Breite des Gesichtsfeldes 44 beträgt im dargestellten Beispiel 0,95.

Die Mediane 52, 54 beziehen sich auf weiße/farblose Stimuli einer geringeren Leuchtdichte/Helligkeit, und die Mediane 56, 58 beziehen sich auf rote Stimuli gleicher Größe und geringerer Leuchtdichte. Hier besitzt die normierte Größe den Wert 0,73.

Während die Figur 2a Daten für ein linkes Auge der normalsichtigen Testperson zeigt, stellt die Figur 2b Daten für das rechte Auge dieser Testperson dar.

Die beiden ganz außenliegenden Mediane 60, 62 schließen ein Gesichtsfeld 64 für weiße/farblose Stimuli einer ersten Leuchtdichte/Helligkeit ein. Die Mediane 66 und 68 (fällt mit 62 zusammen) schließen ein Gesichtsfeld 70 für rote Stimuli ein, die in Bezug auf ihre "Helligkeit" und "Größe" den weißen/farblosen Stimuli des Gesichtsfeldes 64 entsprechen.

Die durch Quotientenbildung erfolgte Normierung der Breite des Gesichtsfeldes 70 auf die Breite des Gesichtsfeldes 64 beträgt im dargestellten Beispiel 0,99.

Die Mediane 72, 74 beziehen sich auf weiße/farblose Stimuli einer geringeren Leuchtdichte/Helligkeit, und die Mediane 76, 78 beziehen sich auf rote Stimuli gleicher Größe und geringerer Leuchtdichte. Hier besitzt die normierte Größe den Wert 0,71.

Die Figur 3a zeigt im Einzelnen der Figur 3 vergleichbare Daten für eine Testperson mit einer deutlichen Protanomalie (Rotschwäche) .

Die beiden ganz außenliegenden Mediane 80, 82 schließen ein Gesichtsfeld 84 für weiße/farblose Stimuli einer ersten Leuchtdichte/Helligkeit ein. Die Mediane 86 und 88 schließen ein Gesichtsfeld 90 für rote Stimuli ein, die in Bezug auf ihre Helligkeit und Größe den weißen/farblosen Stimuli des Gesichtsfeldes 84 entsprechen.

Die durch Quotientenbildung erfolgte Normierung der Breite des Gesichtsfeldes 90 auf die Breite des Gesichtsfeldes 84 beträgt im dargestellten Beispiel 0,79.

Die Mediane 92, 94 beziehen sich auf weiße/farblose Stimuli einer geringeren Leuchtdichte/Helligkeit, und die Mediane 96, 98 beziehen sich auf rote Stimuli gleicher Größe und geringerer Leuchtdichte. Hier besitzt die normierte Größe den Wert 0,22.

Während die Figur 3a Daten für ein linkes Auge der eine deutliche Protanomalie aufweisenden normalsichtigen Testperson zeigt, stellt die Figur 3b Daten für das rechte Auge dieser Testperson dar.

Die beiden ganz außen liegenden Mediane 100, 102 schließen ein Gesichtsfeld 104 für weiße/farblose Stimuli einer ersten Leuchtdichte/Helligkeit ein. Die Mediane 106 und 108 schließen ein Gesichtsfeld 110 für rote Stimuli ein, die in Bezug auf ihre Helligkeit und Größe den weißen/farblosen Stimuli des Gesichtsfeldes 104 entsprechen.

Die durch Quotientenbildung erfolgte Normierung der Breite des Gesichtsfeldes 110 auf die Breite des Gesichtsfeldes 104 beträgt im dargestellten Beispiel 0,88.

Die Mediane 112, 114 beziehen sich auf weiße/farblose Stimuli einer geringeren Leuchtdichte/Helligkeit, und die Mediane 116 beziehen sich auf rote Stimuli gleicher Größe und geringerer Leuchtdichte. Hier besitzt die normierte Größe den Wert 0,57.

Das Darstellen der Stimuli 14 erfolgt für eine vorbestimmte Präsentationsdauer. Wenn innerhalb der Präsentationdauer keine Reaktion der Testperson erfolgt, werden für die betreffende Präsentationsdauer auch keine Daten akquiriert.

Die Messung endet, nachdem die Daten für eine bestimmte Anzahl von Stimuli akquiriert worden sind oder das Messpersonal die Messung von Hand abbricht.

Aus den längs jeweils einer individuellen, hier lediglich beispielsweise horizontal verlaufenden Bahnkurve 34 (akquirierten Daten (insbesondere den Schwellenabständen) wird bevorzugt durch statistische Bewertung, insbesondere bevorzugt durch Medianbildung, für diese Bahnkurve 34 ein Punkt bestimmt, dessen Abstand von der Blickfixierungsmarkierung eine statistisch abgesicherte Entfernung kennzeichnet, die sich dadurch definiert ist, dass die Testperson die Stimuli in kleineren Entfernungen wahrnimmt.

Wenn das Stimuli 14 bei der Bestimmung des Abstandes von der Blickfixierungsmarkierung mit vorgegebener Winkelgeschwindigkeit kontinuierlich entlang einer Bahnkurve 34 bewegt worden ist, liegt zwischen dem Moment der Wahrnehmung und der Betätigung der Eingabevorrichtung 34 (oder einer anderen Reaktion) eine Reaktionszeit der Testperson. Wenn diese Reaktionszeit bei der Bestimmung des Abstandes auf der Bahnkurve 34, bei dem der Stimulus 14 wahrgenommen wurde, nicht berücksichtigt wird, ergibt sich ein systematischer Fehler in Form eines zu kleinen Abstands (Die Bewegung der Stimuli ist in der Regel zur Blickfixierungsmarkierung hin gerichtet. Dieser Fehler würde einer stärkeren Beeinträchtigung der Wahrnehmung entsprechen, als tatsächlich vorliegt. Zur Abhilfe wird die Reaktionszeit der Testperson bestimmt und bei der Bestimmung des genannten Punktes der Bahnkurve berücksichtigt. Die Berücksichtigung erfolgt dadurch, dass der Teil der Bahnkurve, den das Stimuli in der Reaktionszeit zurückgelegt, als Produkt aus Reaktionszeit und Stimuliwinkelgeschwindigkeit bestimmt wird und dass der zunächst ohne Berücksichtigung der Reaktionszeit bestimmte Punkt der Bahnkurve um diesen Teil der Bahnkurve in Richtung zu der initialen Position (, also *entgegen* der Bewegungsrichtung des Stimulus zurück-)verlegt wird.

Bei mehreren Isopteren ist prinzipiell auch eine flächenbezogene oder volumetrische Quantifizierung ("Defektvolumen" möglich):
Zum Beispiel stellt die zwischen den beiden Isopteren 44, 42 liegende Differenzfläche ein Maß für die durch die (hier bewusst verschlechternde) Brille verursachte Änderung der Fähigkeit der Testperson zum Peripheren Sehen dar.

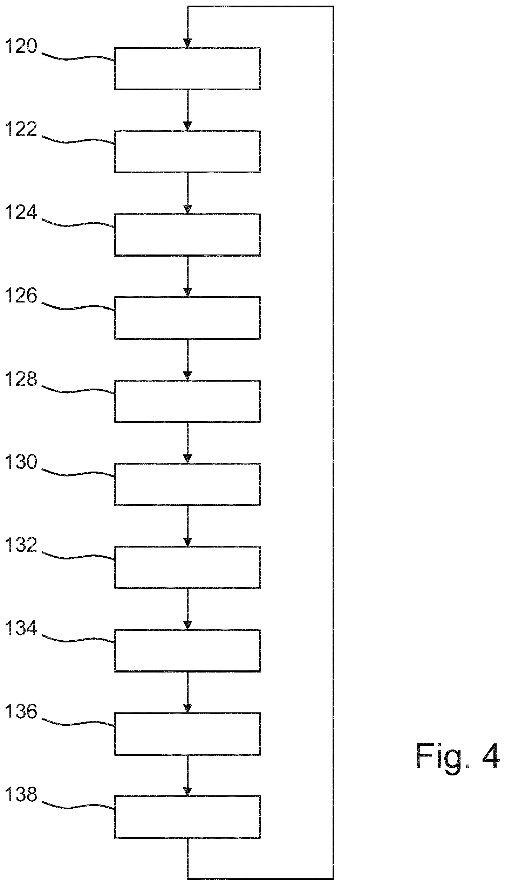

Figur 4 zeigt ein Flussdiagramm als Ausführungsbeispiel eines erfindungsgemäßen Verfahrens. Mit dem im Folgenden verwendeten Begriff des Schrittes kann auch eine Schrittfolge, also ein Unterprogramm gemeint sein. Die Reihenfolge der Schritte/Schrittfolgen muss nicht der bei dem folgenden Ausführungsbeispiel gewählten Reihenfolge entsprechen.

In einem ersten Schritt 120 wird eine Testvorrichtung 10 bereitgestellt, die die Präsentationsfläche 12, eine Eingabevorrichtung 34, eine Blickrichtungserkennungsvorrichtung 28 und ein Steuergerät 22 aufweist.

In einem zweiten Schritt 122 wird die Testperson, deren Daten ermittelt werden sollen, so platziert, dass die Blickfixierungsmarkierung 16 in einem zentralen Bereich des Sichtfeldes der Testperson liegt. Dabei blickt die Testperson bevorzugt geradeaus.

In einem dritten Schritt 124 wird die Testperson angewiesen, ihren Blick auf die Blickfixierungsmarkierung 16 zu richten und nicht von der Blickfixierungsmarkierung 16 zu lösen. Darüber hinaus wird sie dazu angewiesen, eine bestimmte Reaktion zu zeigen, wenn sie einen Stimulus 14 auf der Präsentationsfläche 12 wahrnimmt.

In einem vierten Schritt 126 erfolgt eine Darstellung visueller Stimuli (14) in verschiedenen Positionen einer vorbestimmten Bahnkurve (34) auf der Präsentationsfläche (12).

In einem fünften Schritt 128 erfolgt ein fortwährendes Überwachen der Blickrichtung 26 der Testperson mit der Blickrichtungserkennungsvorrichtung 28.

In einem sechsten Schritt 130 erfolgt fortwährend ein Überwachen der Testperson auf das Zeigen der bestimmten Reaktion. Wenn mit der Blickrichtungserkennungsvorrichtung erkannt wird, dass sich der Blick der Testperson zwischen der Darstellung des Stimulus und der davon ausgelösten Reaktion der Testperson von der Blickfixierungsmarkierung gelöst hat, werden keine Daten akquiriert.

Eine Akquisition von Daten, d.h. ein Erfassen und Speichern von Daten erfolgt nur dann, wenn sich der Blick der Testperson zwischen der Darstellung des Stimulus und der davon ausgelösten Reaktion der Testperson nicht von der Blickfixierungsmarkierung gelöst hat. Bei den Daten handelt es sich bevorzugt um Daten, welche die Position des Stimulus in dem Moment kennzeichnen, in dem die Testperson die Reaktion zeigt.

In einem siebten Schritt 132 erfolgt ein Erfassen und Speichern von Daten, welche die Positionen der visuellen Stimuli 14 in dem Moment kennzeichnen, in dem die Testperson eine Reaktion zeigt.

In einem achten Schritt 134 wird aus den gespeicherten Daten ein Grenzpunkt der Bahnkurve ermittelt, der eine Grenze zwischen Positionen von Stimuli, auf welche die Testperson noch reagiert, und von der Blickfixierungsmarkierung her weiter außen liegenden Positionen von Stimuli, auf welche die Testperson nicht mehr reagiert, markiert.

In einem neunten Schritt 136 wird ein Maß für das Gesichtsfeld der Testperson in Abhängigkeit von dem Grenzpunkt ermittelt, wobei das Maß als ein erstes Maß für erste Stimuli ermittelt wird, die farblos sind oder eine erste Farbe aufweisen, und wobei das Maß als ein zweites Maß für zweite Stimuli ermittelt wird, die eine zweite Farbe aufweisen.

In einem zehnten Schritt 138 werden die Daten, die sich auf eine Wahrnehmung von Farben durch eine Testperson beziehen, durch Normierung des zweiten Maßes auf das erste Maß gebildet. Die Messung wird bevorzugt so oft wiederholt, bis eine vorbestimmte Zahl von Schwellenabständen für jedes Auge und jeden Stimulustyp ermittelt worden ist oder das Messpersonal die Messung von Hand abbricht.

## Patentansprüche

1. Verfahren zur Akquisition von Daten, die sich auf eine Wahrnehmung von Farben durch eine Testperson beziehen, wobei das Verfahren die folgenden Schritte aufweist:
a) Bereitstellen einer Testvorrichtung (10), die wenigstens die folgenden Elemente aufweist:
a1) eine Präsentationsfläche (12), auf der visuelle Stimuli (14) in variablen Positionen und wenigstens eine ortsfeste Blickfixierungsmarkierung (16) darstellbar sind;
a2) eine Eingabevorrichtung (34), mit der Reaktionen der Testperson erfassbar und aufzeichnungsbar sind; und
wobei das Verfahren die folgenden Schritte umfasst:
b) Platzieren der Testperson so, dass die Blickfixierungsmarkierung (16) in einem zentralen Bereich des Sichtfeldes der Testperson liegt;
c) Anweisen der Testperson, ihren Blick auf die Blickfixierungsmarkierung (16) zu richten und nicht von der Blickfixierungsmarkierung (16) zu lösen;
d) Anweisen der Testperson, eine bestimmte Reaktion zu zeigen, wenn sie einen visuellen Stimulus (14) auf der Präsentationsfläche (12) wahrnimmt, wobei die Reaktion auf die Wahrnehmung des visuellen Stimulus' in der Betätigung der Eingabevorrichtung beruht (34);
e) Darstellen visueller Stimuli (14) in verschiedenen Positionen wenigstens einer vorbestimmten Bahnkurve (34) auf der Präsentationsfläche (12);
f) Überwachen der Testperson auf das Zeigen der bestimmten Reaktion;
g) Erfassen und Speichern von Daten, welche die Positionen der visuellen Stimuli (14) in dem Moment kennzeichnen, in dem die Testperson Reaktion zeigt,
aus den gespeicherten Daten erfolgendes Ermitteln eines Grenzpunktes der Bahnkurve, der eine Grenze zwischen Positionen von Stimuli, auf welche die Testperson noch reagiert, und von der Blickfixierungsmarkierung her weiter außen liegenden Positionen von Stimuli, auf welche die Testperson noch nicht reagiert, markiert,
h) Ermitteln eines Maßes für das Gesichtsfeld der Testperson in Abhängigkeit von dem Grenzpunkt, wobei
h1) das Maß als ein erstes Maß für erste Stimuli ermittelt wird, die farblos sind oder eine erste Farbe aufweisen, und wobei
h2) das Maß als ein zweites Maß für zweite Stimuli ermittelt wird, die eine zweite Farbe aufweisen, und wobei die sich auf eine Wahrnehmung von Farben durch eine Testperson beziehenden Daten durch Normierung des zweiten Maßes auf das erste Maß gebildet werden.

2. Verfahren nach Anspruch 1, **dadurch gekennzeichnet, dass** als visueller Stimulus ein Lichtfleck verwendet wird.

3. Verfahren nach Anspruch 1 oder 2, **dadurch gekennzeichnet, dass** die zweite Farbe Rot ist.

4. Verfahren nach einem der Ansprüche 1 bis 3, **dadurch gekennzeichnet, dass** es für verschiedene Leuchtdichten und/oder verschiedene Farben und/oder für verschiedene Größen und/oder verschiedenen Winkelgeschwindigkeiten und/oder verschiedene Helligkeitsänderungen der visuellen Stimuli (14) durchgeführt wird.

5. Verfahren nach einem der vorhergehenden Ansprüche, **dadurch gekennzeichnet, dass** die visuellen Stimuli (14) mit vorgegebener Geschwindigkeit kontinuierlich entlang einer Bahnkurve (34) bewegt werden, die durch die Blickfixierungsmarkierung (16) verläuft.

6. Verfahren nach einem der vorhergehenden Ansprüche, **dadurch gekennzeichnet, dass** der Grenzpunkt aus längs jeweils einer Bahnkurve (34) erfassten Daten durch statistische Bewertung wie Mittelwertbildung oder Medianbildung von Schwellenwertabständen für diese Bahnkurve (34) bestimmt wird, wobei jeder Schwellenwertabstand einen Abstand von der Blickfixierungsmarkierung (16) für diese Bahnkurve (34) eine Entfernung (Exzentrizität) kennzeichnet, bis zu der die betreffende Testperson den visuellen Stimulus (14) erkannt hat.

7. Verfahren nach Anspruch 6, **dadurch gekennzeichnet, dass** bei der Bestimmung der Daten eine Reaktionszeit der Testperson berücksichtigt wird.

8. Verfahren nach Anspruch 7, **dadurch gekennzeichnet, dass** die Berücksichtigung dadurch erfolgt, dass der Teil der Bahnkurve (34), den der visuelle Stimulus (14) in der Reaktionszeit zurückgelegt hat, als Produkt aus Reaktionszeit und der Stimulusgeschwindigkeit bestimmt wird und dass zunächst ohne Berücksichtigung der Reaktionszeit bestimmte Punkte der Bahnkurve (34) als um diesen Teil der Bahnkurve (34) in Richtung zu der initialen Position verlegte Bahnpunkte bestimmt werden.

9. Testvorrichtung (10) zur Akquisition von Daten, die sich auf eine Wahrnehmung von Farben durch eine Testperson beziehen, wobei die Testvorrichtung (10)
- wenigstens eine Präsentationsfläche (12) aufweist, auf der visuelle Stimuli (14) in variablen Positionen darstellbar sind und in der wenigstens eine ortsfeste Blickfixierungsmarkierung (16) darstellbar ist,
- welche Testvorrichtung wenigstens eine zur Erzeugung visueller Stimuli eingerichtete Lichtquelle und
- eine Eingabevorrichtung (34) aufweist, mit der Reaktionen der Testperson erfassbar und aufzeichnungsbar sind,
- die Blickfixierungsmarkierung (16) in einem zentralen Bereich des Sichtfeldes der Testperson zu erzeugen,
wobei die Testvorrichtung dazu eingerichtet ist, visuelle Stimuli (14) in verschiedenen Positionen einer vorbestimmten Bahnkurve (34) auf der Präsentationsfläche (12) zu erzeugen, die Testperson auf das Zeigen der bestimmten Reaktion zu überwachen,
Daten zu erfassen und zu speichern, welche die Positionen der visuellen Stimuli (14) in dem Moment kennzeichnen, in dem die Testperson die Reaktion zeigt,
aus den gespeicherten Daten einen Grenzpunkt der Bahnkurve zu ermitteln, der eine Grenze zwischen Positionen von Stimuli, auf welche die Testperson noch reagiert, und weiter außen liegenden Positionen von Stimuli (14), auf welche die Testperson noch nicht reagiert, markiert,
ein Maß für das Gesichtsfeld der Testperson in Abhängigkeit von dem Grenzpunkt zu ermitteln, und dabei das Maß als ein erstes Maß für erste Stimuli zu ermitteln, die farblos sind oder eine erste Farbe aufweisen, und das Maß als ein zweites Maß für zweite Stimuli zu ermitteln die eine zweite Farbe aufweisen, und
die sich auf eine Wahrnehmung von Farben durch eine Testperson beziehenden Daten durch Normierung des zweiten Maßes auf das erste Maß zu bilden.

10. Testvorrichtung (10) nach Anspruch 9, **dadurch gekennzeichnet, dass** sie dazu eingerichtet ist, ein Verfahren nach einem der Ansprüche 2 bis 8 durchzuführen.

## Claims

1. A method for acquiring data relating to a subject's perception of colors, wherein the method has the following steps:
a) providing a test device (10) having at least the following elements:
a1) a presentation surface (12) on which visual stimuli (14) can be displayed in variable positions and at least one stationary gaze fixation marker (16);
a2) an input device (34) by means of which reactions of the test subject can be detected and recorded; and
the method comprising the following steps:
b) placing the subject in such a manner that the gaze fixation mark (16) is located in a central area of the subject's field of view;
c) instructing the subject to direct his or her gaze to the gaze fixation marker (16) and not to move away from the gaze fixation marker (16);
d) instructing the subject to exhibit a determined response when perceiving a visual stimulus (14) on the presentation surface (12), wherein the response is based on the perception of the visual stimulus' in the actuation of the input device (34);
e) representing visual stimuli (14) in different positions of at least one predetermined track curve (34) on the presentation surface (12);
f) monitoring the subject for showing the determined response;
g) capturing and storing data identifying the positions of the visual stimuli (14) at the moment the subject shows response,
determining from the stored data a boundary point of the track curve which marks a boundary between positions of stimuli to which the subject still responds and positions of stimuli further out from the gaze fixation mark to which the subject does not yet respond,
h) determining a measure of the subject's field of view as a function of the boundary point, wherein
h1) the measure is determined as a first measure for first stimuli that are colorless or have a first color, and wherein
h2) the measure is determined as a second measure for second stimuli having a second color, and wherein the data relating to a perception of colors by a subject is formed by normalizing the second measure to the first measure.

2. The method according to claim 1, **characterized in that** a light spot is used as visual stimulus.

3. The method according to claim 1 or 2, **characterized in that** the second color is red.

4. The method according to any of claims 1 to 3, **characterized in that** it is performed for different luminances and/or different colors and/or for different sizes and/or different angular velocities and/or different brightness changes of the visual stimuli (14).

5. The method of any of the preceding claims, **characterized in that** the visual stimuli (14) are continuously moved at a predetermined speed along a track curve (34) that extends through the gaze fixation marker (16).

6. The method according to one of the preceding claims, **characterized in that** the boundary point is determined from data acquired along a respective track curve (34) by statistical evaluation such as averaging or median formation of threshold distances for said track curve (34), wherein each threshold distance denotes a distance from the gaze fixation marker (16) for said track curve (34) a distance (eccentricity) up to which the subject in question has recognized the visual stimulus (14).

7. The method according to claim 6, **characterized in that** a response time of the test person is taken into account when determining the data.

8. The method according to claim 7, **characterized in that** the consideration is performed by determining the part of the track curve (34) that the visual stimulus (14) has traveled in the reaction time as the product of the reaction time and the stimulus velocity, and that points of the track curve (34) initially determined without consideration of the reaction time are determined as track points displaced about this part of the track curve (34) in the direction toward the initial position.

9. A test device (10) for acquiring data relating to a subject's perception of colors, wherein the test device (10)
- has at least one presentation surface (12) on which visual stimuli (14) can be displayed in variable positions and in which at least one stationary gaze fixation marking (16) can be displayed,
- said test device comprises at least one light source adapted to generate visual stimuli and
- has an input device (34) with which reactions of the test person can be detected and recorded,
- creates the gaze fixation mark (16) in a central area of the subject's field of view,
wherein the test device is configured to generate visual stimuli (14) in various positions of a predetermined track curve (34) on the presentation surface (12), monitor the test subject for showing the determined response,
acquire and store data characterizing the positions of the visual stimuli (14) at the moment when the subject shows the response,
determine from the stored data a boundary point of the track curve which marks a boundary between positions of stimuli to which the test person still reacts and positions of stimuli further out (14) to which the test person does not yet react,
determine a measure of the subject's visual field as a function of the boundary point, thereby determining the measure as a first measure for first stimuli that are colorless or have a first color, and determining the measure as a second measure for second stimuli that have a second color, and forming data relating to a subject's perception of colors by normalizing the second measure to the first measure.

10. The test device (10) according to claim 9, **characterized in that** it is configured to perform a method according to any of claims 2 to 8.

## Revendications

1. Procédé pour l'acquisition de données, qui se réfèrent à une perception de couleurs par une personne soumise à un test, dans lequel le procédé présente les étapes suivantes :
a) la fourniture d'un dispositif de test (10), qui présente au moins les éléments suivants :
al) une surface de présentation (12), sur laquelle des stimuli visuels (14) dans des positions variables et au moins un marquage de fixation du regard (16) fixe peuvent être représentés ;
a2) un dispositif d'entrée (34), avec lequel les réactions de la personne soumise à un test peuvent être acquises et peuvent être enregistrées ; et
dans lequel le procédé comprend les étapes suivantes :
b) le placement de la personne soumise à un test de sorte que le marquage de fixation du regard (16) se situe dans une zone centrale du champ de vision de la personne soumise à un test ;
c) le fait d'indiquer à la personne soumise à un test de diriger son regard sur le marquage de fixation du regard (16) et de ne pas le détacher du marquage de fixation du regard (16) ;
d) le fait d'indiquer à la personne soumise à un test de montrer une réaction définie lorsqu'elle perçoit un stimulus visuel (14) sur la surface de présentation (12), dans lequel la réaction à la perception du stimulus visuel repose sur l'actionnement du dispositif d'entrée (34) ;
e) la représentation de stimuli visuels (14) dans différentes positions au moins d'une trajectoire (34) prédéfinie sur la surface de présentation (12) ;
f) la surveillance de la personne soumise à un test en ce qui concerne la réaction définie qu'elle montre ;
g) l'acquisition et la mise en mémoire de données, lesquelles caractérisent les positions des stimuli visuels (14) au moment où la personne soumise à un test montre une réaction,
la détermination, s'effectuant à partir des données mises en mémoire, d'un point limite de la trajectoire qui marque une limite entre les positions de stimuli auxquels la personne soumise à un test réagit encore et les positions situées davantage à l'extérieur à partir du marquage de fixation du regard de stimuli auxquels la personne soumise à un test ne réagit pas encore,
h) la détermination d'une mesure pour le champ de vision de la personne soumise à un test en fonction du point limite, dans lequel
hl) la mesure est déterminée en tant que première mesure pour des premiers stimuli, qui sont incolores ou présentent une première couleur, et dans lequel
h2) la mesure est déterminée en tant que deuxième mesure pour des deuxièmes stimuli, qui présentent une deuxième couleur, et
dans lequel les données se référant à une perception de couleurs par une personne soumise à un test sont formées par normalisation de la deuxième mesure sur la première mesure.

2. Procédé selon la revendication 1, **caractérisé en ce qu'**une tache lumineuse est utilisée comme stimulus visuel.

3. Procédé selon la revendication 1 ou 2, **caractérisé en ce que** la deuxième couleur est le rouge.

4. Procédé selon l'une quelconque des revendications 1 à 3, **caractérisé en ce qu'**il est mis en oeuvre pour différentes luminances et/ou différentes couleurs et/ou pour différentes grandeurs et/ou différentes vitesses angulaires et/ou différents changements de luminosité des stimuli visuels (14).

5. Procédé selon l'une quelconque des revendications précédentes, **caractérisé en ce que** les stimuli visuels (14) sont déplacés à une vitesse prédéfinie de manière continue le long d'une trajectoire (34), qui s'étend à travers le marquage de fixation du regard (16).

6. Procédé selon l'une quelconque des revendications précédentes, **caractérisé en ce que** le point limite est défini à partir de données acquises le long de respectivement une trajectoire (34) par évaluation statistique telle que la formation de moyenne ou formation de médiane d'écarts de valeur seuil pour cette trajectoire (34), dans lequel chaque écart de valeur seuil caractérise un écart par rapport à au marquage de fixation du regard (16) pour cette trajectoire (34) un éloignement (excentricité) jusqu'auquel la personne soumise à un test concernée a détecté le stimulus visuel (14).

7. Procédé selon la revendication 6, **caractérisé en ce que** lors de la définition des données un temps de réaction de la personne soumise à un test est pris en compte.

8. Procédé selon la revendication 7, **caractérisé en ce que** la prise en compte s'effectue par le fait que la partie de la trajectoire (34) que le stimulus visuel (14) a parcourue dans le temps de réaction est définie comme produit du temps de réaction et de la vitesse de stimulus et que les points de la trajectoire (34) définis tout d'abord sans prise en compte du temps de réaction sont définis en tant que points de trajectoire déplacés de cette partie de la trajectoire (34) en direction de la position initiale.

9. Dispositif de test (10) pour l'acquisition de données, qui se réfèrent à une perception de couleurs par une personne soumise à un test, dans lequel le dispositif de test (10)
- présente au moins une surface de présentation (12), sur laquelle des stimuli visuels (14) dans des positions variables peuvent être représentés et dans laquelle au moins un marquage de fixation du regard (16) fixe peut être représenté et,
- lequel dispositif de test présente au moins une source de lumière conçue pour la production de stimuli visuels et
- un dispositif d'entrée (34), avec lequel les réactions de la personne soumise à un test peuvent être acquises et peuvent être enregistrées,
- pour produire le marquage de fixation du regard (16) dans une zone centrale du champ de vision de la personne soumise à un test,
dans lequel le dispositif de test est conçu pour produire des stimuli visuels (14) dans différentes positions d'une trajectoire (34) prédéfinie sur la surface de présentation (12), pour surveiller la personne soumise à un test en ce qui concerne la réaction définie qu'elle montre,
pour acquérir et pour mettre en mémoire des données, lesquelles caractérisent les positions des stimuli visuels (14) au moment où la personne soumise à un test montre la réaction,
pour déterminer à partir des données mises en mémoire un point limite de la trajectoire qui marque une limite entre les positions de stimuli auxquels la personne soumise à un test réagit encore et les positions situées davantage à l'extérieur de stimuli (14) auxquels la personne soumise à un test ne réagit pas encore,
pour déterminer une mesure pour le champ de vision de la personne soumise à un test en fonction du point limite, et ainsi pour déterminer la mesure en tant que première mesure pour des premiers stimuli, qui sont incolores ou présentent une première couleur, et pour déterminer la mesure comme une deuxième mesure pour des deuxièmes stimuli qui présentent une deuxième couleur, et
pour former les données se référant à une perception de couleurs par une personne soumise à un test par normalisation de la deuxième mesure sur la première mesure.

10. Dispositif de test (10) selon la revendication 9, **caractérisé en ce qu'**il est conçu pour mettre en oeuvre un procédé selon l'une quelconque des revendications 2 à 8.
